# FASCICULE DE BREVET EUROPEEN

(11) **EP 1 713 543 B1**
(45) Date de publication et mention de la délivrance du brevet: **11.11.2009**
(21) Numéro de dépôt: 05717556.4
(22) Date de dépôt: 04.02.2005
(51) Int. Cl.: A61P 35/00, A61K 31/437

(54) **UTILISATION DE DERIVES D'INDOLIZINE 1,2,3 SUBSTITUES POUR LE TRAITEMENT DE MALADIES LIEES A UNE ANGIOGENESE PATHOLOGIQUE CHOROIDIENNE**
VERWENDUNG VON SUBSTITUIERTEN 1,2,3-INDOLIZIN-DERIVATEN ZUR BEHANDLUNG VON KRANKHEITEN IM ZUSAMMENHANG MIT EINER PATHOLOGISCHEN ANGIOGENESE
USE OF SUBSTITUTED 1,2,3 INDOLIZINE DERIVATIVES FOR TREATING DISEASES ASSOCIATED WITH A PATHOLOGICAL ANGIOGENESIS

(30) Priorité: 05.02.2004 FR 0401094
(43) Date de publication de la demande: 25.10.2006
(73) Titulaire: Sanofi-Aventis, 75013 Paris (FR)
(72) Inventeur: BADORC, Alain, F-31120 Roquettes (FR); BONO, Françoise, F-31300 Toulouse (FR); BORDES, Marie-Françoise, F-31860 Labarthe sur Leze (FR); FOIDART, Jean-Michel, B-4870 Trooz (BE); GUILLO, Nathalie, F-31000 Toulouse (FR); NOEL, Agnès, B-4653 Bolland (BE); RAKIC, Jean-Marie, B-5370 Flostoy (BE)
(74) Mandataire: Romanowski, Caroline
(86) Numéro de dépôt international: PCT/FR2005/000253
(87) Numéro de publication internationale: WO 2005/082457

(56) Documents cités:
- WO-A-03/084956
- ROUSSEAU BENOÎT ET AL: "Involvement of fibroblast growth factors in choroidal angiogenesis and retinal vascularization." EXPERIMENTAL EYE RESEARCH. AUG 2003, vol. 77, no. 2, août 2003 (2003-08), pages 147-156, XP002296587 ISSN: 0014-4835 cité dans la demande
- SOUBRANE G ET AL: "Basic fibroblast growth factor experimentally induced choroidal angiogenesis in the minipig." CURRENT EYE RESEARCH. MAR 1994, vol. 13, no. 3, mars 1994 (1994-03), pages 183-195, XP009036634 ISSN: 0271-3683
- ROUSSEAU B ET AL: "Neural and angiogenic defects in eyes of transgenic mice expressing a dominant-negative FGF receptor in the pigmented cells." EXPERIMENTAL EYE RESEARCH. OCT 2000, vol. 71, no. 4, octobre 2000 (2000-10), pages 395-404, XP002296589 ISSN: 0014-4835
- TOBE TAKAO ET AL: "Targeted disruption of the FGF2 gene does not prevent choroidal neovascularization in a murine model" AMERICAN JOURNAL OF PATHOLOGY, vol. 153, no. 5, novembre 1998 (1998-11), pages 1641-1646, XP001183277 ISSN: 0002-9440 cité dans la demande
- OZAKI H ET AL: "Basic fibroblast growth factor is neither necessary nor sufficient for the development of retinal neovascularization." AMERICAN JOURNAL OF PATHOLOGY. SEP 1998, vol. 153, no. 3, septembre 1998 (1998-09), pages 757-765, XP001183611 ISSN: 0002-9440
- YAMADA HARUHIKO ET AL: "Cell injury unmasks a latent proangiogenic phenotype in mice with increased expression of FGF2 in the retina" JOURNAL OF CELLULAR PHYSIOLOGY, vol. 185, no. 1, octobre 2000 (2000-10), pages 135-142, XP009036644 ISSN: 0021-9541 cité dans la demande

## Description

Il a été montré *in vitro* et *in vivo* que plusieurs facteurs de croissance participent à la mise en place d'un déséquilibre "proangiogène", conduisant à la prolifération incontrôlée des cellules endothéliales, observée dans les phénomènes de néovascularisation. Le FGF2 ou bFGF (Fibroblast Growth Factor 2 ou b) est le premier et le mieux caractérisé. FGF2 est une protéine de 18000 D qui induit la prolifération, la migration et la production de protéases par les cellules endothéliales en culture et la néovascularisation in vivo. FGF2 interagit avec les cellules endothéliales par l'intermédiaire de 2 classes de récepteurs, les récepteurs de haute affinité à activité tyrosine kinase (FGFRs) et les récepteurs de basse affinité de type héparan sulfate protéoglycane (HSPGs) situé à la surface des cellules et dans les matrices extracellulaires. Alors que le rôle paracrine du FGF2 sur les cellules endothéliales est largement décrit, le FGF2 pourrait également intervenir sur ces cellules à travers un processus autocrine. Ainsi, FGF2 et ses récepteurs représentent des cibles très pertinentes pour les thérapies visant à inhiber les processus d'angiogénèse (Keshet E, Ben-Sasson SA.,. J. Clin. lnvest, (1999), vol 501, pp104-1497 ; Presta M, Rusnati M, Dell'Era P, Tanghetti E, Urbinati C, Giuliani R et al, New York: Plenum Publishers, (2000), pp7-34, Billottet C, Janji B, Thiery J.P., Jouanneau J, Oncogene, (2002) vol 21, pp8128-8139).

Une étude récente a montré, via un modèle d'étude expérimental réalisé sur un oeil de souris, l'implication des FGFs sur la néovascularisation (Rousseau et al. « lnvolvement of Fibroblasts Growth Factors in choroïdal angiogenesis and retinal vascularization », Experimental Eye Research, Laboratoire des Mécanismes Moléculaires de l'angiogénèse, INSERM EML01-13, Université de Bordeaux, France, 13 mai 2003, p.147-156). Le rôle précis des FGFs et de leurs récepteurs dans les processus d'angiogénèse pathologique de l'oeil, et en particulier de la choroïde, reste cependant mal connu et laisse la voie à des hypothèses parfois divergentes (Tobe et al., « Targeted disruption of the FGF2 gene does not prevent choroïdal neovascularization in a murine model », Am. J. Pathol. 153, 1641-1646, 1998 / Yamada et al., « Cell injury unmasks a latent proangiogenic phenotype in mice with increased expression of FGF2 in the retina, J. Cell Physiol. 185, 135-142, 2000).

La demande de brevet internationale WO 03/084956 décrit des dérivés, inhibiteurs de FGFs utiles, entre autre, dans le traitement de pathologies dans lesquelles l'angiogénèse semble affecter de manière importante la progression, telles que par exemple, les maladies inflammatoires chroniques comme l'arthrite rhumatoïde ou les IBD (Inflammatory Bowel Diseases). La demande de brevet internationale WO 03/084956 décrit également que de tels composés sont utiles pour le traitement de maladies consécutives à des complications vasculaires du diabète, comme la rétinopathie diabétique dans laquelle les vaisseaux sanguins de la rétine peuvent se fissurer ou se boucher.

Il a été maintenant trouvé que certains dérivés d'indolizine 1, 2, 3 substitués, qui sont des antagonistes des récepteurs aux FGFs, dits FGFRs, sont utiles pour le traitement de maladies liées à une angiogénèse pathologique choroïdienne. De manière surprenante, ces composés sont aussi actifs lorsque administrés par voie orale que par voie locale, notamment par voie intraoculaire. La présente invention concerne donc l'utilisation de dérivés d'indolizine 1, 2, 3 substitués, pour la préparation de médicaments utiles pour le traitement de maladies liées à une angiogénèse pathologique choroïdienne, dérivés correspondants à la formule générale 1 suivante : 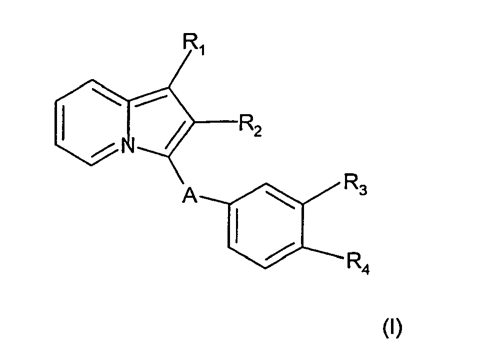 dans laquelle :
**R₁** représente un radical hydroxy, un radical alcoxy linéaire ou ramifié de 1 à 5 atomes de carbone, un radical carboxy, un radical alcoxycarbonyle de 2 à 6 atomes de carbone ou un radical de formule :
   - NR₅R₆
   - -NH-SO₂-Alk
   - -NH-SO₂-Ph
   - -NH-CO-Ph
   - -N(Alk)-CO-Ph
   - -NH-CO-NH-Ph
   - -NH-CO-Alk
   - -NH-CO₂₋Alk
   - -O-(CH₂)ₙ-cAlk
   - -O-Alk-COOR₇
   - -O-Alk-O-R₈
   - -O-Alk-OH
   - -O-Alk-C(NH₂):NOH
   - -O-Alk-NR₅R₆
   - -O-Alk-CN
   - -O-(CH₂)ₙ-Ph
   - -O-Alk-CO-NR₅R₆
   - -CO-NH-(CH₂)ₘ-COOR₇
   - -CO-NH-Alk
   dans lesquelles
   - Alk représente un radical alkyle ou un radical alkylène linéaire ou ramifié de 1 à 5 atomes de carbone,
   - cAlk représente un radical cycloalkyle de 3 à 6 atomes de carbone,
   - n représente un nombre entier de 0 à 5,
   - m représente un nombre entier de 1 à 5,
   - R₅ et R₆ identiques ou différents représentent chacun un atome d'hydrogène, un radical alkyle linéaire ou ramifié de 1 à 5 atomes de carbone ou un radical benzyle,
   - R₇ représente un atome d'hydrogène ou un radical alkyle de 1 à 5 atomes de carbone,
   - R₈ représente un radical alkyle de 1 à 5 atomes de carbone ou un radical -CO-Alk,
   - Ph représente un radical phényle éventuellement substitué par un ou plusieurs atomes d'halogène, par un ou plusieurs radicaux alcoxy de 1 à 5 atomes de carbone, par un ou plusieurs radicaux carboxy ou par un ou plusieurs radicaux alcoxycarbonyle de 2 à 6 atomes de carbone,
**R₂** représente un atome d'hydrogène, un radical alkyle de 1 à 5 atomes de carbone, un radical d'halogenure d'alkyle de 1 à 5 atomes de carbone comportant 3 à 5 atomes d'halogène, un radical cycloalkyle de 3 à 6 atomes de carbone ou un radical phényle éventuellement substitué par un ou plusieurs atomes d'halogène, par un ou plusieurs radicaux alcoxy de 1 à 5 atomes de carbone, un ou plusieurs radicaux carboxy ou par un ou plusieurs radicaux alcoxycarbonyle de 2 à 6 atomes de carbone,
**A** représente un radical -CO-, -SO- ou -SO₂-,
**R₃** et **R₄** identiques ou différents représentent chacun un atome d'hydrogène, un radical alcoxy de 1 à 5 atomes de carbone, un radical amino, un radical carboxy, un radical alcoxycarbonyle de 2 à 6 atomes de carbone, un radical hydroxy, un radical nitro, un radical hydroxyamino, un radical de formule
   - -Alk-COOR₇
   - -NR₅R₆
   - -NH-Alk-COOR₇
   - -NH-COO-Alk
   - -N(R₁₁)-SO₂-Alk-NR₉R₁₀
   - -N(R₁₁)-SO₂-Alk
   - -N(R₁₁)-Alk-NR₅R₆
   - -N(R₁₁)-CO-Alk-NR₉R₁₀
   - -N(R₁₁)-CO-Alk
   - -N(R₁₁)-CO-CF₃
   - -NH-Alk-HetN
   - -O-Alk-NR₉R₁₀
   - -O-Alk-CO-NR₅R₆
   - -O-Alk-HetN
dans lesquels n, m, Alk, R₅, R₆, et R₇, ont la signification donnée précédemment pour R₁ ; et
**R₉** et **R₁₀** identiques ou différents représentent chacun un atome d'hydrogène ou un radical alkyle de 1 à 5 atomes de carbone,
**R₁₁** représente un atome d'hydrogène ou un radical -Alk-COOR₁₂ où R₁₂ représente un atome d'hydrogène, un radical alkyle de 1 à 5 atomes de carbone ou un radical benzyle,
**HetN** représente un hétérocycle à 5 ou 6 chaînons comportant au moins un atome d'azote et éventuellement un autre hétéroatome choisi parmi l'azote et l'oxygène ;
ou bien **R₃** et **R₄** forment ensemble un hétérocycle insaturé de 5 à 6 chaînons, à condition toutefois que lorsque R₃ représente un radical alcoxy et R₄ représente un radical -O-Alk-NR₉R₁₀ ou un radical hydroxy, R₁ ne représente pas un radical alcoxy, éventuellement sous la forme de l'un de leurs sels pharmaceutiquement acceptables.

On préfère utiliser les composés de formule générale I sans laquelle :
**R₁** représente un radical hydroxy, un radical alcoxy linéaire ou ramifié de 1 à 5 atomes de carbone, un radical carboxy, un radical alcoxycarbonyle de 2 à 6 atomes de carbone ou un radical de formule :
   - -NR₅R₆
   - -NH-SO₂-Alk
   - -NH-SO₂-Ph
   - -NH-CO-Ph
   - -N(Alk)-CO-Ph
   - -NH-CO-NH-Ph
   - -NH-CO-Alk
   - -NH-CO₂-Alk
   - -O-(CH₂)ₙ-cAlk
   - -O-Alk-COOR₇
   - -O-Alk-O-R₈
   - -O-Alk-OH
   - -O-Alk-NR₅R₆
   - -O-Alk-CN
   - -O-(CH₂)ₙ-Ph
   - -O-Alk-CO-NR₅R₆
   - -CO-NH-(CH₂)ₘ-COOR₇
   - -CO-NH-Alk
   dans lesquelles
   - Alk représente un radical alkyle ou un radical alkylène linéaire ou ramifié de 1 à 5 atomes de carbone,
   - cAlk représente un radical cycloalkyle de 3 à 6 atomes de carbone,
   - n représente un nombre entier de 0 à 5,
   - m représente un nombre entier de 1 à 5,
   - R₅ et R₆ identiques ou différents représentent chacun un atome d'hydrogène, un radical alkyle linéaire ou ramifié de 1 à 5 atomes de carbone ou un radical benzyle,
   - R₇ représente un atome d'hydrogène ou un radical alkyle de 1 à 5 atomes de carbone,
   - R₈ représente un radical alkyle de 1 à 5 atomes de carbone ou un radical -CO-Alk
   - Ph représente un radical phényle éventuellement substitué par un ou plusieurs atomes d'halogène, par un ou plusieurs radicaux alcoxy de 1 à 5 atomes de carbone, un ou plusieurs radicaux carboxy ou par un
   ou plusieurs radicaux alcoxycarbonyle de 2 à 6 atomes de carbone,
**R₂** représente un radical alkyle de 1 à 5 atomes de carbone, un radical trifluorométhyle, un radical cycloalkyle de 3 à 6 atomes de carbone ou un radical phényle éventuellement substitué par un ou plusieurs atomes d'halogène, par un
ou plusieurs radicaux alcoxy de 1 à 5 atomes de carbone, par un ou plusieurs radicaux carboxy ou par un ou plusieurs radicaux alcoxycarbonyle de 2 à 6 atomes de carbone,
**A** représente un radical -CO-, -SO₂-,
**R₃** et **R₄** identiques ou différents représentent chacun un atome d'hydrogène, un radical alcoxy de 1 à 5 atomes de carbone, un radical amino, un radical carboxy, un radical alcoxycarbonyle de 2 à 6 atomes de carbone, un radical nitro, un radical hydroxyamino, un radical de formule
   - -Alk-COOR₇
   - -NR₅R₆
   - -NH-Alk-COOR₇
   - -NH-COO-Alk
   - -N(R₁₁)-SO₂-Alk-NR₉R₁₀
   - -N(R₁₁-SO₂-Alk
   - -N(R₁₁)-Alk-NR₅R₆
   - -N(R₁₁)-CO-Alk-NR₉R₁₀
   - -N(R₁₁)-CO-Alk
   - -N(R₁₁)-CO-CF₃
   - -NH-Alk-HetN
      dans lesquels n, m, Alk, R₅, R₆, et R₇, ont la signification donnée précédemment pour R₁ ; et
**R₉** et **R₁₀** identiques ou différents représentent chacun un atome d'hydrogène ou un radical alkyle de 1 à 5 atomes de carbone,
**R₁₁** représente un atome d'hydrogène ou un radical -Alk-COOR₁₂ où R₁₂ représente un atome d'hydrogène, un radical alkyle de 1 à 5 atomes de carbone ou un radical benzyle,
**HetN** représente un hétérocycle à 5 ou 6 chaînons comportant au moins un atome d'azote et éventuellement un autre hétéroatome choisi parmi l'azote et l'oxygène, éventuellement sous la forme de l'un de leurs sels pharmaceutiquement acceptables.

L'utilisation des composés de formule générale 1 dans laquelle :
**R₁** représente un radical alcoxy de 1 à 5 atomes de carbone, un radical carboxy, un radical -O-Alk-COOH dans laquelle Alk représente un radical alkylène linéaire ou ramifié de 1 à 5 atomes de carbone, un radical de formule -O-Alk-Ph dans laquelle Alk représente un radical alkylène de 1 à 5 atomes de carbone et Ph représente un radical phényle éventuellement substitué par un ou plusieurs atomes d'halogène ou par un ou plusieurs radicaux alcoxy de 1 à 5 atomes de carbone ou par un ou plusieurs radicaux carboxy, un radical de formule -NH-CO-Ph, un radical de formule -NH-SO₂-Ph ou un radical de formule -NH-CO-NH-Ph,
**R₂** représente un radical alkyle de 1 à 5 atomes de carbone,
**A** représente un radical -CO-,
**R₃** et **R₄** différents représentent chacun un atome d'hydrogène, un radical alcoxy de 1 à 5 atomes de carbone, un radical amino, un radical carboxy un radical alcoxycarbonyle de 2 à 6 atomes de carbone,
éventuellement sous la forme de l'un de ses sels pharmaceutiquement acceptables, est particulièrement préférée.

On préfère utiliser plus particulièrement les composés de formule 1 choisis parmi :
- (4-amino -3-méthoxy phényl) (1-méthoxy 2-méthyl indolizin-3-yl) méthanone
- acide 3-(4-amino -3-méthoxy benzoyl) 2-méthyl indolizin-1-yl carboxylique
- acide 2-{[3-(4-amino-3-méthoxybenzoyl) 2-méthyl indolizin-1-yl]oxy} acétique
- (4-amino -3-méthoxy phényl) {1-[(4-chlorobenzyl)oxy] 2-méthylindolizin-3-yl} méthanone
- (4-amino-3-méthoxy phényl) {1-[(3-méthoxybenzyl)oxy] 2-méthylindolizin-3-yl} méthanone
- acide 4-({[3-(4-amino-3-méthoxy benzoyl) 2-méthyl indolizin-1-yl]oxy} méthyl) benzoique
- acide 3-(4-carboxybenzoyl) 2-méthyl indolizin-1-yl carboxylique
- 3-[(1-méthoxy-2-méthyl indolizin-3-yl carbonyl] benzoate de méthyle
- acide 4-[(1-méthoxy-2-méthyl indolizin-3-yl) carbonyl] benzoïque
- acide 2-amino-5-[(1-méthoxy-2-méthylindolizin-3-yl) carbonyl] benzoïque
- acides 2-amino-5-({1-[(3-méthoxybenzoyl)amino]-2-méthylindolizin-3-yl} carbonyl) benzoïque
- acide 2-amino-5-({2-méthyl-1-[(3,4,5-triméthoxybenzoyl)amino] indolizin-3-yl} carbonyl) benzoïque
- acide 2-amino-5-({1-{[(3-méthoxyphényl)sulfonyl]amino}-2-méthylindolizin-3-yl} carbonyl) benzoïque,
éventuellement sous la forme de l'un de ses sels pharmaceutiquement acceptables.

L'angiogénèse pathologique choroïdienne est un processus de génération de nouveaux vaisseaux capillaires de la choroïde. La choroïde est une membrane de l'oeil très vascularisée : tout un réseau de fins capillaires assurent la nutrition de l'iris et de la rétine à la périphérie de laquelle elle se trouve. L'angiogénèse pathologique choroïdienne est majoritairement due à la mise en place d'un déséquilibre "pro angiogène", mais aussi à des altérations de la membrane de Bruch, située sous la rétine. Ainsi, les capillaires choroïdiens prolifèrent de façon incontrôlée et, via une défectuosité dans la membrane de Bruch, envahissent l'espace sous rétinien (Lafaut et al., Br. J. Ophtalmol. 84, 239-243).

Parmi les maladies liées à une angiogénèse pathologique choroïdienne et notamment consécutive à une anomalie de la membrane de Bruch, on peut citer la dégénérescence maculaire liée à l'âge (DMLA), la myopie forte (Quaranta et al., Graefe's Arch. Clin. Exp. Ophtalmol., 238, 101-103), le pseudo xanthome, le syndrome d'histoplasmose présumée, la toxoplasmose, la sarcoïdose et la maladie de Behcet.

Ainsi, selon un de ses aspects, la présente invention concerne l'utilisation de dérivés d'indolizine 1,2,3 substitués de formule générale 1, pour la préparation de médicaments utiles pour le traitement de maladies liées à une angiogénèse pathologique choroïdienne, telles que la dégénérescence maculaire lié à l'âge (DMLA), la myopie forte, le pseudo xanthome, le syndrome d'histoplasmose présumée, la toxoplasmose, la sarcoïdose, la maladie de Behcet.

Selon un autre de ses aspects, la présente invention a pour objet une composition pharmaceutique contenant au moins un principe actif correspondant à un composés de formule I ou un de ses sels pharmaceutiquement acceptables, éventuellement en association avec un ou plusieurs excipients inertes et appropriés, utile pour le traitement de maladies liées à une angiogénèse pathologique choroïdienne.

La présente invention a en particulier pour objet une composition pharmaceutique contenant au moins un principe actif correspondant à un composé de formule 1 choisi parmi :
- (4-amino-3-méthoxy phényl) (1-méthoxy 2-méthyl indolizin-3-yl) méthanone
- acide 3-(4-amino -3-méthoxy benzoyl) 2-méthyl indolizin-1-yl carboxylique
- acide 2-{[3-(4-amino-3-méthoxybenzoyl) 2-méthyl indolizin-1-yl] oxy} acétique
- (4-amino-3-méthoxy phényl) {1-[(4-chlorobenzyl)oxy] 2-mëthylindolizin-3-yl} méthanone
- (4-amino-3-méthoxy phényl) {1-[(3-méthoxybenzyl)oxy] 2-méthylindolizin-3-yl} méthanone
- acide 4-({[3-(4-amino-3-méthoxy benzoyl) 2-méthyl indolizin-1-yl]oxy} méthyl) benzoique
- acide 3-(4-carboxybenzoyl) 2-méthyl indolizin-1-yl carboxylique
- 3-[(1-méthoxy-2-méthyl indolizin-3-yl carbonyl] benzoate de méthyle
- acide 4-[(1-méthoxy-2-méthyl indolizin-3-yl)carbonyl] benzoïque
- acide 2-amino-5-[(1-méthoxy-2-méthylindolizin-3-yl)carbonyl] benzoïque
- acide 2-amino-5-({1-[(3-méthoxybenzoyl)amino]-2-méthylindolizin-3-yl} carbonyl) benzoïque
- acide 2-amino-5-({2-méthyl-1-[(3,4,5-triméthoxybenzoyl)amino] indolizin-3-yl} carbonyl) benzoïque
- acide 2-amino-5-({1-{[(3-méthoxyphényl)sulfonyl] amino}-2-méthylindolizin-3-yl} carbonyl) benzoïque,
éventuellement en association avec un ou plusieurs excipients inertes et appropriés.

Lesdits excipients sont choisis selon la forme pharmaceutique et le mode d'administration souhaitée : orale, intraoculaire ou locale.

Les compositions pharmaceutiques de la présente invention sont administrées de préférence par voie orale ou intraoculaire.

Les compositions pharmaceutiques de la présente invention sont administrées de manière particulièrement préférée, par voie orale.

Dans les compositions pharmaceutiques des la présente invention pour l'administration orale, les principes actifs peuvent être administrés sous forme unitaire d'administration, en mélange avec des supports pharmaceutiques classiques. Les formes unitaires d'administration appropriées comprennent par exemple les comprimés éventuellement sécables, les gélules, les poudres, les granules et les solutions ou suspensions orales.

Lorsqu'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues.
On peut enrober les comprimés de saccharose ou d'autres matières appropriées
ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants ou des agents de mise en suspension, comme la polyvinylpyrrolidone, de même qu'avec des édulcorants ou des correcteurs du goût.

Le principe actif peut être formulé également sous forme de microcapsules, éventuellement avec un ou plusieurs supports ou additifs.

Dans les compositions pharmaceutiques selon la présente invention, le principe actif peut être aussi éventuellement sous forme de complexe d'inclusion dans des cyclodextrines, leurs éthers ou leurs esters.

Les compositions pharmaceutiques à administration locale peuvent être sous forme de différentes formulations ophtalmiques. Elles peuvent contenir conjointement au principe actif des préservatifs ophthalmologiquement acceptables, des surfactants, des agents de viscosité, des agents qui favorisent la pénétration du principe actif, des tampons, de chlorure de sodium ainsi que de l'eau afin d'obtenir des solutions ou suspensions stériles à usage ophtalmologique.

Les solutions ophtalmiques peuvent être préparées en dissolvant les principes actifs dans des solutions tampons isotoniques. Ces solutions peuvent contenir des surfactants afin de faciliter la dissolution du principe actif. Afin de permettre une meilleure application, les solutions ophtalmiques peuvent également contenir un agent d'épaississement tels que l'hydroxymethylcellulose, l'hydroxyethylcellulose, l'hydroxypropyl-méthylcellulose ou le polyvinylpyrrolidone.

Pour préparer des pommades ophtalmiques stériles, le principe actif est combiné avec un préservatif dans un véhicule approprié, par exemple la lanoline liquide. Des gels stériles peuvent être préparés en mettant en suspension le principe actif dans une base hydrophile contenant du carbopol-940 ou d'autres composés analogues.

Pour une application locale, les compositions pharmaceutiques selon l'invention sont de préférence sous forme de solutions ou des suspensions ayant un pH de 4 à 8. La concentration du principe actif doit être de 0,0001% à 5% p/p, de préférence 0,0001 à 1%. On peut administrer selon la prescription médicale une à deux gouttes de ces compositions 1 à 4 fois par jour.

La quantité de principe actif à administrer dépend, comme toujours, du degré de progression de la maladie ainsi que de l'âge et du poids du patient.

En particulier, les compositions pharmaceutiques administrables par voie orale selon l'invention, contiennent des doses recommandées de principe actif comprises entre 1 et 900 mg/kg/jour.

De préférence, les compositions pharmaceutiques administrables par voie orale selon l'invention, contiennent des doses recommandées de principe actif comprises entre 3 et 300 mg/kg/jour.

Les compositions pharmaceutiques administrable par voie orale selon l'invention, contiennent de façon particulièrement préférée des doses recommandées de principe actif comprises entre 1 et 100 mg/kg/jour.

L'exemple suivant, donné à titre non limitatif, illustre la présente invention.

### Exemple : Induction au laser de néovascularisation choroïdienne.

### 1. Matériel et méthode

Des souris C57BI6 ont été utilisées dans ces expériences. Un modèle de néovascularisation choroïdienne induite au laser (4 impacts par oeil autour de la papille) a été appliqué sur les animaux comme décrit par Tobe et al Am. J. Pathol, 1998).
Au jour 14 après l'induction et après un contrôle angiographique permettant d'estimer le pourcentage de lésions développant une néovascularisation, les animaux sont sacrifiés et les globes énucléés pour analyse histologique.
Les souris sont traitées avec un des composés de formule I durant les derniers 7 jours par voie orale. La dose choisie pour le monohydrate du sel sodique d'acide 2-amino-5-[(1-méthoxy-2-méthylindolizin-3-yl)carbonyl]benzoïque est de 30 mg/kg/jour. Le véhicule utilisé est une solution de méthylcellulose dans l'eau à O.6%.
Quatre souris ont utilisées pour le lot contrôle et six souris pour le lot traité.

La taille de la réaction néovasculaire est estimée par évaluation morphométrique de l'épaisseur à l'aide d'un système informatisé d'analyse d'images à partir de coupes congelées. Ces dernières sont soit, simplement colorées à l'hématoxyline, soit examinées en immunofluorescence en utilisant un anticorps permettant de localiser les structures vasculaires (anti-CD31).

L'estimation se fait par la mesure du rapport B/C entre, d'une part, l'EPR et le sommet de la réaction néovasculaire (« B »), et d'autre part l'épaisseur de la couche choroïdienne intacte adjacente (« C »). Ce système de quantification a été préféré à une mesure de surfaces de lésions car il est indépendant de l'orientation des sections histologiques.

### 2. Résultats :

Les angiographies effectuées au jour 14 ont montré l'induction de néovaisseaux dans 72% des lésions créées au laser dans les souris contrôle.
L'analyse histologique et l'immunohistochimie effectuée à l'aide de l'anticorps anti-CD31 ont confirmé la présence de capillaires néoformés au niveau des zones montrant une diffusion importante de fluorescéine à l'angiographie dans le groupe contrôle. Par contre, les animaux traités avec le composé n'ont présenté qu'un épaississement modéré de la choriocapillaire au niveau des impacts, sans signe évident de néovascularisation.
La quantification de la réaction induite par le laser effectuée par la mesure du rapport B/C, a montré une réduction significative de l'ordre de 50% de la réaction (p<0.001) chez les souris traitées avec le composé comparativement aux souris contrôles.

Ces résultats confirment le grand intérêt de l'utilisation de composés de formule 1 pour la préparation de médicaments utiles pour le traitement de maladies liées à une angiogénèse pathologique choroïdienne

## Revendications

1. Utilisation de dérivés d'indolizine 1,2,3 substitués, de formule générale I suivants : 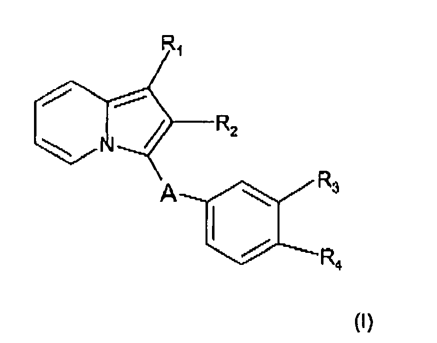 dans laquelle :
**R₁** représente un radical hydroxy, un radical alcoxy linéaire ou ramifié de 1 à 5 atomes de carbone, un radical carboxy, un radical alcoxycarbonyle de 2 à 6 atomes de carbone ou un radical de formule :
• -NR₅R₆
• -NH-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₈
• -O-Alk-OH
• -O-Alk-C(NH₂):NOH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
dans lesquelles
• Alk représente un radical alkyle ou un radical alkylène linéaire ou ramifié de 1 à 5 atomes de carbone,
• cAlk représente un radical cycloalkyle de 3 à 6 atomes de carbone,
• n représente un nombre entier de 0 à 5,
• m représente un nombre entier de 1 à 5, ,
• R₅ et R₆ identiques ou différents représentent chacun un atome d'hydrogène, un radical alkyle linéaire ou ramifié de 1 à 5 atomes de carbone ou un radical benzyle,
• R₇ représente un atome d'hydrogène ou un radical alkyle de 1 à 5 atomes de carbone,
• R₈ représente un radical alkyle de 1 à 5 atomes de carbone ou un radical -CO-Alk,
• Ph représente un radical phényle éventuellement substitué par un ou plusieurs atomes d'halogène, par un ou plusieurs radicaux alcoxy de 1 à 5 atomes de carbone, par un ou plusieurs radicaux carboxy ou par un ou plusieurs radicaux alcoxycarbonyle de 2 à 6 atomes de carbone,
**R₂** représente un atome d'hydrogène, un radical alkyle de 1 à 5 atomes de carbone, un radical d'halogenure d'alkyle de 1 à 5 atomes de carbone comportant 3 à 5 atomes d'halogène, un radical cycloalkyle de 3 à 6 atomes de carbone ou un radical phényle éventuellement substitué par un ou plusieurs atomes d'halogène, par un ou plusieurs radicaux alcoxy de 1 à 5 atomes de carbone, un ou plusieurs radicaux carboxy ou par un ou plusieurs radicaux alcoxycarbonyle de 2 à 6 atomes de carbone,
**A** représente un radical -CO-, -SO- ou -SO₂-,
**R₃** et **R₄** identiques ou différents représentent chacun un atome d'hydrogène, un radical alcoxy de 1 à 5 atomes de carbone, un radical amino, un radical carboxy, un radical alcoxycarbonyle de 2 à 6 atomes de carbone, un radical hydroxy, un radical nitro, un radical hydroxyamino, un radical de formule
• -Alk-COOR₇
• -NR₅R₆
• -NH-Alk-COOR₇
• -NH-COO-Alk
• -N(R₁₁)-SO₂-Alk-NR₉R₁₀
• -N(R₁₁)-SO₂-Alk
• -N(R₁₁)-Alk-NR₅R₆
• -N(R₁₁)-CO-Alk-NR₉R₁₀
• -N(R₁₁)-CO-Alk
• N(R₁₁)-CO-CF₃
• -NH-Alk-HetN
• -O-Alk-NR₉R₁₀
• -O-Alk-CO-NR₅R₆
• -O-Alk-HetN
dans lesquels n, m, Alk, R₅, R₆, et R₇, ont la signification donnée précédemment pour R₁ ; et
**R₉** et **R₁₀** identiques ou différents représentent chacun un atome d'hydrogène ou un radical alkyle de 1 à 5 atomes de carbone,
**R₁₁** représente un atome d'hydrogène ou un radical -Alk-COOR₁₂ où R₁₂ représente un atome d'hydrogène, un radical alkyle de 1 à 5 atomes de carbone ou un radical benzyle,
**HetN** représente un hétérocycle à 5 ou 6 chaînons comportant au moins un atome d'azote et éventuellement un autre hétéroatome choisi parmi l'azote et l'oxygène;
ou bien **R₃** et **R₄** forment ensemble un hétérocycle insaturé de 5 à 6 chaînons,
à condition toutefois que lorsque R₃ représente un radical alcoxy et R₄ représente un radical - O-Alk-NR₉R₁₀ ou un radical hydroxy, R₁ ne représente pas un radical alcoxy,
éventuellement sous la forme de l'un de leurs sels pharmaceutiquement acceptables,
pour la préparation de médicaments pour le traitement de maladies liées à une angiogénèse pathologique choroïdienne, choisies parmi la dégénérescence maculaire liée à l'âge (DMLA), la myopie forte, le pseudo xanthome, le syndrome d'histoplasmose présumée la toxoplasmose, la sarcoïdose et la maladie de Behcet.

2. Utilisation selon la revendication 1, de composés de formule générale 1 dans laquelle:
**R₁** représente un radical hydroxy, un radical alcoxy linéaire ou ramifié de 1 à 5 atomes de carbone, un radical carboxy, un radical alcoxycarbonyle de 2 à 6 atomes de carbone ou un radical de formule :
• -NR₅R₆
• -NH-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₆
• -O-Alk-OH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
dans lesquelles
• Alk représente un radical alkyle ou un radical alkylène linéaire
ou ramifié de 1 à 5 atomes de carbone,
• cAlk représente un radical cycloalkyle de 3 à 6 atomes de carbone,
• n représente un nombre entier de 0 à 5.
• m représente un nombre entier de 1 à 5,
• R₅ et R₆ identiques ou différents représentent chacun un atome d'hydrogène, un radical alkyle linéaire ou ramifié de 1 à 5 atomes de carbone ou un radical benzyle,
• R₇ représente un atome d'hydrogène ou un radical alkyle de 1 à 5 atomes de carbone,
• R₈ représente un radical alkyle de 1 à 5 atomes de carbone ou un radical -CO-Alk
• Ph représente un radical phényle éventuellement substitué par un ou plusieurs atomes d'halogène, par un ou plusieurs radicaux alcoxy de 1 à 5 atomes de carbone, un ou plusieurs radicaux carboxy ou par un ou plusieurs radicaux alcoxycarbonyle de 2 à 6 atomes de carbone,
**R₂** représente un radical alkyle de 1 à 5 atomes de carbone, un radical trifluorométhyle, un radical cycloalkyle de 3 à 6 atomes de carbone ou un radical phényle éventuellement substitué par un ou plusieurs atomes d'halogène, par un ou plusieurs radicaux alcoxy de 1 à 5 atomes de carbone, par un ou plusieurs radicaux carboxy ou par un ou plusieurs radicaux alcoxycarbonyle de 2 à 6 atomes de carbone,
**A** représente un radical -CO-, -SO₂-,
**R₃** et **R₄** identiques ou différents représentent chacun un atome d'hydrogène, un radical alcoxy de 1 à 5 atomes de carbone, un radical amino, un radical carboxy, un radical alcoxycarbonyle de 2 à 6 atomes de carbone, un radical nitro, un radical hydroxyamino, un radical de formule
• -Alk-COOR₇
• -NR₅R₆
• -NH-Alk-COOR₇
• -NH-COO-Alk
• -N(R₁₁)-SO₂-Alk-NR₉R₁₀
• -N(R₁₁)-SO₂-Alk
• -N(R₁₁)-Alk-NR₅R₆
• -N(R₁₁)-CO-Alk-NR₉R₁₀
• -N(R₁₁)-CO-Alk
• -N(R₁₁)-CO-CF₃
• -NH-Alk-HetN
dans lesquels n, m, Alk, R₅, R₆, et R₇, ont la signification donnée précédemment pour R₁ ; et
**R₉** et **R₁₀** identiques ou différents représentent chacun un atome d'hydrogène ou un radical alkyle de 1 à 5 atomes de carbone,
**R₁₁** représente un atome d'hydrogène ou un radical -Alk-COOR₁₂ où R₁₂ représente un atome d'hydrogène, un radical alkyle de 1 à 5 atomes de carbone ou un radical benzyle,
**HetN** représente un hétérocycle à 5 ou 6 chaînons comportant au moins un atome d'azote et éventuellement un autre hétéroatome choisi parmi l'azote et l'oxygène, éventuellement sous la forme de l'un de leurs sels pharmaceutiquement acceptables.

3. Utilisation selon l'une quelconque des revendications 1 à 2, de composés de formule générale 1, dans laquelle:
**R₁** représente un radical alcoxy de 1 à 5 atomes de carbone, un radical carboxy, un radical - O-Alk-COOH dans laquelle Alk représente un radical alkylène linéaire ou ramifié de 1 à 5 atomes de carbone, un radical de formule -O-Alk-Ph dans laquelle Alk représente un radical alkylène de 1 à 5, atomes de carbone et Ph représente un radical phényle éventuellement substitué par un ou plusieurs atomes d'halogène ou par un ou plusieurs radicaux alcoxy de 1 à 5 atomes de carbone ou par un ou plusieurs radicaux carboxy, un radical de formule -NH-CO-Ph, un radical de formule -NH-SO₂-Ph ou un radical de formule -NH-CO-NH-Ph,
**R₂** représente un radical alkyle de 1 à 5 atomes de carbone,
**A** représente un radical -CO-,
**R₃** et **R₄** différents représentent chacun un atome d'hydrogène, un radical alcoxy de 1 à 5 atomes de carbone, un radical amino, un radical carboxy un radical alcoxycarbonyle de 2 à 6 atomes de carbone,
éventuellement sous la forme de l'un de ses sels pharmaceutiquement acceptables.

4. Utilisation selon l'une quelconque des revendications 1 à 3, de composés de formule générale 1 choisis parmi :
- (4-amino-3-méthoxy phényl) (1-méthoxy 2-méthyl indolizin-3-yl) méthanone
- acide 3-(4-amino-3-méthoxy benzoyl) 2-méthyl indolizin-1-yl carboxylique
- acide 2-{[3-(4-amino-3-méthoxybenzoyl) 2-méthyl indolizin-1-yl] oxy} acétique
- (4-amino-3-méthoxy phényl) {1-[(4-chlorobenzyl)oxy] 2-méthylindolizin-3-yl} méthanone
- (4-amino-3-méthoxy phényl) {1-[(3-méthoxybenzyl)oxy] 2-méthylindolizin-3-yl} méthanone
- acide 4-({[3-(4-amino-3-méthoxy benzoyl) 2-méthyl indolizin-1-yl]oxy} méthyl) benzoique
- acide 3-(4-carboxybenzoyl) 2-méthyl indolizin-1-yl carboxylique
- 3-[(1-méthoxy-2-méthyl indolizin-3-yl carbonyl] benzoate de méthyle
- acide 4-[(1-méthoxy-2-méthyl indolizin-3-yl)carbonyl] benzoïque
- acide 2-amino-5-[(1-méthoxy-2-méthylindolizin-3-yl)carbonyl] benzoïque
- acide 2-amino-5-({1-[(3-méthoxybenzoyl)amino]-2-méthylindolizin-3-yl} carbonyl) benzoïque
- acide 2-amino-5-({2-méthyl-1-[(3,4,5-triméthoxybenzoyl)amino]indolizin-3-yl} carbonyl) benzoïque
- acide 2-amino-5-({1-{[(3-méthoxyphényl)sulfonyl]amino}-2-méthylindolizin-3-yl} carbonyl) benzoïque,
éventuellement sous la forme de l'un de ses sels pharmaceutiquement acceptables.

## Claims

1. Use of 1,2,3-substituted indolizine derivatives of following general formula (I): 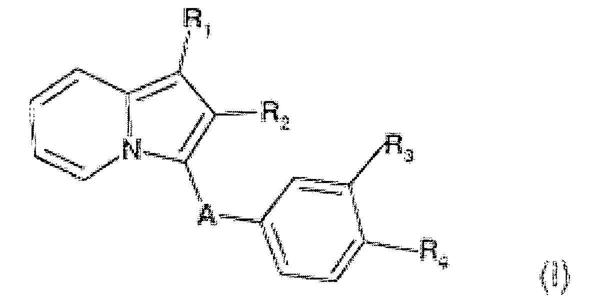 in which:
**R₁** represents a hydroxyl radical, a linear or branched alkoxy radical of 1 to 5 carbon atoms, a carboxyl radical, an alkoxycarbonyl radical of 2 to 6 carbon atoms or a radical of formula:
• -NR₅R₆
• -NH-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₈
• -O-Alk-OH
• -O-Alk-C(NH₂):NOH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
in which
• Alk represents an alkyl radical or an alkylene radical which is linear or branched and which has from 1 to 5 carbon atoms,
• cAlk represents a cycloalkyl radical of 3 to 6 carbon atoms,
• n represents an integer from 0 to 5,
• m represents an integer from 1 to 5,
• R₅ and R₆, which are identical or different, each represent a hydrogen atom, a linear or branched alkyl radical of 1 to 5 carbon atoms or a benzyl radical,
• R₇ represents a hydrogen atom or an alkyl radical of 1 to 5 carbon atoms,
• R₈ represents an alkyl radical of 1 to 5 carbon atoms or a -CO-Alk radical,
• Ph represents a phenyl radical optionally substituted by one or more halogen atoms, by one or more alkoxy radicals of 1 to 5 carbon atoms, by one or more carboxyl radicals or by one or more alkoxycarbonyl radicals of 2 to 6 carbon atoms,
**R₂** represents a hydrogen atom, an alkyl radical of 1 to 5 carbon atoms, an alkyl halide radical of 1 to 5 carbon atoms comprising 3 to 5 halogen atoms, a cycloalkyl radical of 3 to 6 carbon atoms or a phenyl radical optionally substituted by one or more halogen atoms, by one or more alkoxy radicals of 1 to 5 carbon atoms, by one or more carboxyl radicals or by one or more alkoxycarbonyl radicals of 2 to 6 carbon atoms,
**A** represents a -CO-, -SO- or -SO₂- radical,
**R₃** and **R₄**, which are identical or different, each represent a hydrogen atom, an alkoxy radical of 1 to 5 carbon atoms, an amino radical, a carboxyl radical, an alkoxycarbonyl radical of 2 to 6 carbon atoms, a hydroxyl radical, a nitro radical, a hydroxyamino radical, a radical of formula
• -Alk-COOR₇
• -NR₅R₆
• -NH-Alk-COOR₇
• -NH-COO-Alk
• -N(R₁₁)-SO₂-Alk-NR₉R₁₀
• -N(R₁₁)-SO₂-Alk
• -N(R₁₁)-Alk-NR₅R₆
• -N(R₁₁)-CO-Alk-NR₉R₁₀
• -N(R₁₁)-CO-Alk
• -N(R₁₁)-CO-CF₃
• -NH-Alk-HetN
• -O-Alk-NR₉R₁₀
• -O-Alk-CO-NR₅R₆
• -O-Alk-HetN
in which n, m, Alk, R₅, R₆ and R₇ have the meaning given above for R₁; and
**R₉** and **R₁₀**, which are identical or different, each represent a hydrogen atom or an alkyl radical of 1 to 5 carbon atoms,
**R₁₁** represents a hydrogen atom or an -Alk-COOR₁₂ radical where R₁₂ represents a hydrogen atom, an alkyl radical of 1 to 5 carbon atoms or a benzyl radical,
**HetN** represents a 5- or 6-membered heterocycle comprising at least one nitrogen atom and optionally one other heteroatom chosen from nitrogen and oxygen;
or else **R₃** and **R₄** together form an unsaturated 5- to 6-membered heterocycle, provided, however, that, when R₃ represents an alkoxy radical and R₄ represents an -O-Alk-NR₉R₁₀ radical or a hydroxyl radical, R₁ does not represent an alkoxy radical,
optionally in the form of one of their pharmaceutically acceptable salts,
in the preparation of medicaments for the treatment of diseases related to pathological choroidal angiogenesis, chosen from age-related macular degeneration (AMD), severe myopia, pseudoxanthoma, presumed histoplasmosis syndrome, toxoplasmosis, sarcoidosis and Behcet's disease.

2. Use according to Claim 1, of compounds of general formula (I) in which:
**R₁** represents a hydroxyl radical, a linear or branched alkoxy radical of 1 to 5 carbon atoms, a carboxyl radical, an alkoxycarbonyl radical of 2 to 6 carbon atoms or a radical of formula:
• -NR₅R₆
• -NH-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₈
• -O-Alk-OH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
in which
• Alk represents an alkyl radical or an alkylene radical which is linear or branched and which has from 1 to 5 carbon atoms,
• cAlk represents a cycloalkyl radical of 3 to 6 carbon atoms,
• n represents an integer from 0 to 5,
• m represents an integer from 1 to 5,
• R₅ and R₆, which are identical or different, each represent a hydrogen atom, a linear or branched alkyl radical of 1 to 5 carbon atoms or a benzyl radical,
• R₇ represents a hydrogen atom or an alkyl radical of 1 to 5 carbon atoms,
• R₈ represents an alkyl radical of 1 to 5 carbon atoms or a -CO-Alk radical,
• Ph represents a phenyl radical optionally substituted by one or more halogen atoms, by one or more alkoxy radicals of 1 to 5 carbon atoms, by one or more carboxyl radicals or by one or more alkoxycarbonyl radicals of 2 to 6 carbon atoms,
**R₂** represents an alkyl radical of 1 to 5 carbon atoms, a trifluoromethyl radical, a cycloalkyl radical of 3 to 6 carbon atoms or a phenyl radical optionally substituted by one or more halogen atoms, by one or more alkoxy radicals of 1 to 5 carbon atoms, by one or more carboxyl radicals or by one or more alkoxycarbonyl radicals of 2 to 6 carbon atoms,
**A** represents a -CO- or -SO₂- radical,
**R₃** and **R₄**, which are identical or different, each represent a hydrogen atom, an alkoxy radical of 1 to 5 carbon atoms, an amino radical, a carboxyl radical, an alkoxycarbonyl radical of 2 to 6 carbon atoms, a nitro radical, a hydroxyamino radical, a radical of formula
• -Alk-COOR₇
• -NR₅R₆
• -NH-Alk-COOR₇
• -NH-COO-Alk
• -N(R₁₁)-SO₂-Alk-NR₉R₁₀
• -N(R₁₁)-SO₂-Alk
• -N(R₁₁)-Alk-NR₅R₆
• -N(R₁₁)-CO-Alk-NR₉R₁₀
• -N(R₁₁)-CO-Alk
• -N(R₁₁)-CO-CF₃
• -NH-Alk-HetN
in which n, m, Alk, R₅, R₆ and R₇ have the meaning given above for R₁; and
**R₉** and **R₁₀**, which are identical or different, each represent a hydrogen atom or an alkyl radical of 1 to 5 carbon atoms,
**R₁₁** represents a hydrogen atom or an -Alk-COOR₁₂ radical where R₁₂ represents a hydrogen atom, an alkyl radical of 1 to 5 carbon atoms or a benzyl radical,
**HetN** represents a 5- or 6-membered heterocycle comprising at least one nitrogen atom and optionally one other heteroatom chosen from nitrogen and oxygen, optionally in the form of one of their pharmaceutically acceptable salts.

3. Use according to either one of Claims 1 and 2, of compounds of general formula (I) in which:
**R₁** represents an alkoxy radical of 1 to 5 carbon atoms, a carboxyl radical, an -O-Alk-COOH radical in which Alk represents a linear or branched alkylene radical of 1 to 5 carbon atoms, a radical of formula -O-Alk-Ph in which Alk represents an alkylene radical of 1 to 5 carbon atoms and Ph represents a phenyl radical optionally substituted by one or more halogen atoms or by one or more alkoxy radicals of 1 to 5 carbon atoms or by one or more carboxyl radicals, a radical of formula -NH-CO-Ph, a radical of formula -NH-SO₂-Ph or a radical of formula -NH-CO-NH-Ph,
**R₂** represents an alkyl radical of 1 to 5 carbon atoms,
**A** represents a -CO- radical,
**R₃** and **R**_{**4**,} which are different, each represent a hydrogen atom, an alkoxy radical of 1 to 5 carbon atoms, an amino radical, a carboxyl radical or an alkoxycarbonyl radical of 2 to 6 carbon atoms,
optionally in the form of one of their pharmaceutically acceptable salts.

4. Use according to any one of Claims 1 to 3, of compounds of general formula (I) chosen from:
- (4-amino-3-methoxyphenyl)(1-methoxy-2-methylindolizin-3-yl)methanone
- 3-(4-amino-3-methoxybenzoyl)-2-methylindolizin-1-ylcarboxylic acid
- 2-{[3-(4-amino-3-methoxybenzoyl)-2-methylindolizin-1-yl]oxy}acetic acid
- (4-amino-3-methoxyphenyl){1-[(4-chlorobenzyl)oxy]-2-methylindolizin-3-yl}-methanone
- (4-amino-3-methoxyphenyl){1-[(3-methoxybenzyl)oxy]-2-methylindolizin-3-yl}- methanone
- 4-({[3-(4-amino-3-methoxybenzoyl)-2-methylindolizin-1-yl]oxy}methyl)benzoic acid
- 3-(4-carboxybenzoyl)-2-methylindolizin-1-ylcarboxylic acid
- methyl 3-[(1-methoxy-2-methylindolizin-3-yl)carbonyl]benzoate
- 4-[(1-methoxy-2-methylindolizin-3-yl)carbonyl]benzoic acid
- 2-amino-5-[(1-methoxy-2-methylindolizin-3-yl)carbonyl]benzoic acid
- 2-amino-5-({1-[(3-methoxybenzoyl)amino]-2-methylindolizin-3-yl}carbonyl)-benzoic acid
- 2-amino-5-({2-methyl-1-[(3,4,5-trimethoxybenzoyl)amino]indolizin-3-yl}carbonyl)benzoic acid
- 2-amino-5-({1-{[(3-methoxyphenyl)sulfonyl]amino}-2-methylindolizin-3-yl}-carbonyl)benzoic acid,
optionally in the form of one of their pharmaceutically acceptable salts.

## Patentansprüche

1. Verwendung von 1,2,3-substituierten Indolizinderivaten der folgenden allgemeinen Formel I: 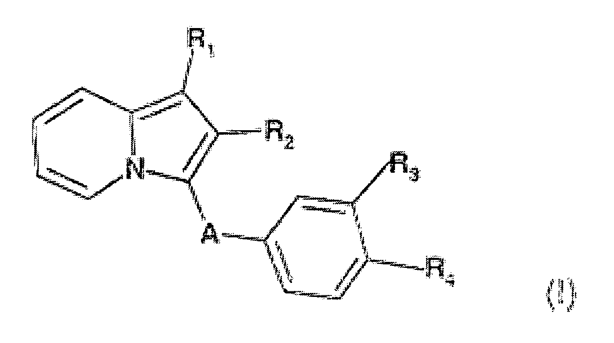 worin:
**R₁** für einen Hydroxylrest, einen linearen oder verzweigten Alkoxyrest mit 1 bis 5 Kohlenstoffatomen, einen Carboxylrest, einen Alkoxycarbonylrest mit 2 bis 6 Kohlenstoffatomen oder einen Rest der Formel:
• -NR₅R₆
• -NH-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₈
• -O-Alk-OH
• -O-Alk-C(NH₂):NOH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
steht, worin
• Alk für einen Alkylrest oder einen Alkylenrest, der linear oder verzweigt ist und 1 bis 5 Kohlenstoffatome aufweist, steht,
• cAlk für einen Cycloalkylrest mit 3 bis 6 Kohlenstoffatomen steht,
• n für eine ganze Zahl von 0 bis 5 steht,
• m für eine ganze Zahl von 1 bis 5 steht,
• R₅ und R₆ gleich oder verschieden sind und jeweils für ein Wasserstoffatom, einen linearen oder verzweigten Alkylrest mit 1 bis 5 Kohlenstoffatomen oder einen Benzylrest stehen,
• R₇ für ein Wasserstoffatom oder einen Alkylrest mit 1 bis 5 Kohlenstoffatomen steht,
• R₈ für einen Alkylrest mit 1 bis 5 Kohlenstoffatomen oder einen -CO-Alk-Rest steht,
• Ph für einen Phenylrest steht, der gegebenenfalls durch ein oder mehrere Halogenatome, einen oder mehrere Alkoxyreste mit 1 bis 5 Kohlenstoffatomen, einen oder mehrere Carboxylreste oder einen oder mehrere Alkoxycarbonylreste mit 2 bis 6 Kohlenstoffatomen substituiert ist,
**R₂** für ein Wasserstoffatom, einen Alkylrest mit 1 bis 5 Kohlenstoffatomen, einen Alkylhalogenidrest mit 1 bis 5 Kohlenstoffatomen, der 3 bis 5 Halogenatome umfaßt, einen Cycloalkylrest mit 3 bis 6 Kohlenstoffatomen oder einen Phenylrest, der gegebenenfalls durch ein oder mehrere Halogenatome, einen oder mehrere Alkoxyreste mit 1 bis 5 Kohlenstoffatomen, einen oder mehrere Carboxylreste oder einen oder mehrere Alkoxycarbonylreste mit 2 bis 6 Kohlenstoffatomen substituiert ist, steht,
**A** für einen -CO-, -SO- oder -SO₂-Rest steht,
**R₃** und **R₄** gleich oder verschieden sind und jeweils für ein Wasserstoffatom, einen Alkoxyrest mit 1 bis 5 Kohlenstoffatomen, einen Aminorest, einen Carboxylrest, einen Alkoxycarbonylrest mit 2 bis 6 Kohlenstoffatomen, einen Hydroxylrest, einen Nitrorest, einen Hydroxyaminorest oder einen Rest der Formel
• -Alk-COOR₇
• -NR₅R₆
• -NH-Alk-COOR₇
• -NH-COO-Alk
• -N(R₁₁)-SO₂-Alk-NR₉R₁₀
• -N(R₁₁)-SO₂-Alk
• -N(R₁₁)-Alk-NR₅R₆
• -N(R₁₁)-CO-Alk-NR₉R₁₀
• -N(R₁₁)-CO-Alk
• -N(R₁₁)-CO-CF₃
• -NH-Alk-HetN
• -O-Alk-NR₉R₁₀
• -O-Alk-CO-NR₅R₆
• -O-Alk-HetN
stehen, worin n, m, Alk, R₅, R₆ und R₇ die oben für R₁ angegebene Bedeutung besitzen und
**R₉** und **R₁₀** gleich oder verschieden sind und jeweils für ein Wasserstoffatom oder einen Alkylrest mit 1 bis 5 Kohlenstoffatomen stehen,
R₁₁ für ein Wasserstoffatom oder einen -Alk-COOR₁₂-Rest steht, wobei R₁₂ für ein Wasserstoffatom, einen Alkylrest mit 1 bis 5 Kohlenstoffatomen oder einen Benzylrest steht,
**HetN** für einen 5- oder 6-gliedrigen Heterocyclus mit mindestens einem Stickstoffatom und gegebenenfalls einem weiteren unter Stickstoff und Sauerstoff ausgewählten Heteroatom steht;
oder **R₃** und **R₄** auch gemeinsam einen ungesättigten 5- oder 6-gliedrigen Heterocyclus bilden,
jedoch mit der Maßgabe, daß dann, wenn R₃ für einen Alkoxyrest steht und R₄ für einen -O-Alk-NR₉R₁₀-Rest oder einen Hydroxylrest steht, R₁ nicht für einen Alkoxyrest steht,
gegebenenfalls in Form eines ihrer pharmazeutisch akzeptablen Salze,
bei der Herstellung von Arzneimitteln zur Behandlung von Erkrankungen, die mit pathologischer Aderhaut-Angiogenese verbunden sind, ausgewählt unter altersbedingter Makuladegeneration (AMD), schwerer Myopie, Pseudoxanthom, vermutetem Histoplasmosesyndrom, Toxoplasmose, Sarkoidose und
Behcet-Krankheit.

2. Verwendung nach Anspruch 1 von Verbindungen der allgemeinen Formel I, worin:
**R₁** für einen Hydroxylrest, einen linearen oder verzweigten Alkoxyrest mit 1 bis 5 Kohlenstoffatomen, einen Carboxylrest, einen Alkoxycarbonylrest mit 2 bis 6 Kohlenstoffatomen oder einen Rest der Formel:
• -NR₅R₆
• -NH-SO₂-Alk
• -NH-SO₂-Ph
• -NH-CO-Ph
• -N(Alk)-CO-Ph
• -NH-CO-NH-Ph
• -NH-CO-Alk
• -NH-CO₂-Alk
• -O-(CH₂)ₙ-cAlk
• -O-Alk-COOR₇
• -O-Alk-O-R₈
• -O-Alk-OH
• -O-Alk-NR₅R₆
• -O-Alk-CN
• -O-(CH₂)ₙ-Ph
• -O-Alk-CO-NR₅R₆
• -CO-NH-(CH₂)ₘ-COOR₇
• -CO-NH-Alk
steht, worin
• Alk für einen Alkylrest oder einen Alkylenrest, der linear oder verzweigt ist und 1 bis 5 Kohlenstoffatome aufweist, steht,
• cAlk für einen Cycloalkylrest mit 3 bis 6 Kohlenstoffatomen steht,
• n für eine ganze Zahl von 0 bis 5 steht,
• m für eine ganze Zahl von 1 bis 5 steht,
• R₅ und R₆ gleich oder verschieden sind und jeweils für ein Wasserstoffatom, einen linearen oder verzweigten Alkylrest mit 1 bis 5 Kohlenstoffatomen oder einen Benzylrest stehen,
• R₇ für ein Wasserstoffatom oder einen Alkylrest mit 1 bis 5 Kohlenstoffatomen steht,
• R₈ für einen Alkylrest mit 1 bis 5 Kohlenstoffatomen oder einen -CO-Alk-Rest steht,
• Ph für einen Phenylrest steht, der gegebenenfalls durch ein oder mehrere Halogenatome, einen oder mehrere Alkoxyreste mit 1 bis 5 Kohlenstoffatomen, einen oder mehrere Carboxylreste oder einen oder mehrere Alkoxycarbonylreste mit 2 bis 6 Kohlenstoffatomen substituiert ist,
**R₂** für einen Alkylrest mit 1 bis 5 Kohlenstoffatomen, einen Trifluormethylrest, einen Cycloalkylrest mit 3 bis 6 Kohlenstoffatomen oder einen Phenylrest, der gegebenenfalls durch ein oder mehrere Halogenatome, einen oder mehrere Alkoxyreste mit 1 bis 5 Kohlenstoffatomen, einen oder mehrere Carboxylreste oder einen oder mehrere Alkoxycarbonylreste mit 2 bis 6 Kohlenstoffatomen substituiert ist, steht,
**A** für einen -CO- oder -SO₂-Rest steht,
**R₃** und **R₄** gleich oder verschieden sind und jeweils für ein Wasserstoffatom, einen Alkoxyrest mit 1 bis 5 Kohlenstoffatomen, einen Aminorest, einen Carboxylrest, einen Alkoxycarbonylrest mit 2 bis 6 Kohlenstoffatomen, einen Nitrorest, einen Hydroxyaminorest oder einen Rest der Formel
• -Alk-COOR₇
• -NR₅R₆
• -NH-Alk-COOR₇
• -NH-COO-Alk
• -N(R₁₁)-SO₂-Alk-NR₉R₁₀
• -N(R₁₁)-SO₂-Alk
• -N(R₁₁)-Alk-NR₅R₆
• -N(R₁₁)-CO-Alk-NR₉R₁₀
• -N(R₁₁)-CO-Alk
• -N(R₁₁)-CO-CF₃
• -NH-Alk-HetN
stehen, worin n, m, Alk, R₅, R₆ und R₇ die oben für R₁ angegebene Bedeutung besitzen und
**R₉** und R₁₀ gleich oder verschieden sind und jeweils für ein Wasserstoffatom oder einen Alkylrest mit 1 bis 5 Kohlenstoffatomen stehen,
R₁₁ für ein Wasserstoffatom oder einen -Alk-COOR₁₂-Rest steht, wobei R₁₂ für ein Wasserstoffatom, einen Alkylrest mit 1 bis 5 Kohlenstoffatomen oder einen Benzylrest steht,
**HetN** für einen 5- oder 6-gliedrigen Heterocyclus mit mindestens einem Stickstoffatom und gegebenenfalls einem weiteren unter Stickstoff und
Sauerstoff ausgewählten Heteroatom steht, gegebenenfalls in Form eines ihrer pharmazeutisch akzeptablen Salze.

3. Verwendung nach einem der Ansprüche 1 bis 2 von Verbindungen der allgemeinen Formel I, worin:
**R₁** für einen Alkoxyrest mit 1 bis 5 Kohlenstoffatomen, einen Carboxylrest, einen -O-Alk-COOH-Rest, worin Alk für einen linearen oder verzweigten Alkylenrest mit 1 bis 5 Kohlenstoffatomen steht, einen Rest der Formel -O-Alk-Ph, worin Alk für einen Alkylenrest mit 1 bis 5 Kohlenstoffatomen steht und Ph für einen Phenylrest, der gegebenenfalls durch ein oder mehrere Halogenatome, einen oder mehrere Alkoxyreste mit 1 bis 5 Kohlenstoffatomen oder einen oder mehrere Carboxylreste substituiert ist, steht, einen Rest der Formel -NH-CO-Ph, einen Rest der Formel -NH-SO₂-Ph oder einen Rest der Formel -NH-CO-NH-Ph steht,
**R₂** für einen Alkylrest mit 1 bis 5 Kohlenstoffatomen steht,
**A** für einen -CO-Rest steht,
**R₃** und **R₄** verschieden sind und jeweils für ein Wasserstoffatom, einen Alkoxyrest mit 1 bis 5 Kohlenstoffatomen, einen Aminorest, einen Carboxylrest oder einen Alkoxycarbonylrest mit 2 bis 6 Kohlenstoffatomen stehen,
gegebenenfalls in Form eines ihrer pharmazeutisch akzeptablen Salze.

4. Verwendung nach einem der Ansprüche 1 bis 3 von Verbindungen der allgemeinen Formel I, ausgewählt unter:
- (4-Amino-3-methoxyphenyl)(1-methoxy-2-methylindolizin-3-yl)methanon
- 3-(4-Amino-3-methoxybenzoyl)-2-methylindolizin-1-ylcarbonsäure
- 2-{[3-(4-Amino-3-methoxybenzoyl)-2-methylindolizin-1-yl]oxy}essigsäure
- (4-Amino-3-methoxyphenyl){1-[(4-chlorbenzyl)-oxy]-2-methylindolizin-3-yl}-methanon
- (4-Amino-3-methoxyphenyl){1-[(3-methoxybenzyl)-oxy]-2-methylindolizin-3-yl}-methanon
- 4-({[3-(4-Amino-3-methoxybenzoyl)-2-methylindolizin-1-yl]oxy}methyl)benzoesäure
- 3-(4-Carboxybenzoyl)-2-methylindolizin-1-yl-carbonsäure
- 3-[(1-Methoxy-2-methylindolizin-3-yl)carbonyl]-benzoesäuremethylester
- 4-[(1-Methoxy-2-methylindolizin-3-yl)carbonyl]-benzoesäure
- 2-Amino-5-[(1-methoxy-2-methylindolizin-3-yl)-carbonyl]benzoesäure
- 2-Amino-5-({1-[(3-methoxybenzoyl)amino]-2-methylindolizin-3-yl}carbonyl)benzoesäure
- 2-Amino-5-({2-methyl-1-[(3,4,5-trimethoxybenzoyl)amino]indolizin-3-yl}carbonyl)benzoesäure
- 2-Amino-5-({1-{[(3-methoxyphenyl)sulfonyl]-amino}-2-methylindolizin-3-yl}-carbonyl)-benzoesäure,
gegebenenfalls in Form eines ihrer pharmazeutisch akzeptablen Salze.
